# EUROPEAN PATENT APPLICATION

(11) **EP 3 957 255 A1**
(43) Date of publication of application: **23.02.2022**
(21) Application number: 20209818.2
(22) Date of filing: 25.11.2020
(51) Int. Cl.: A61B 17/08, A61B 17/12, A61B 17/122, A61B 17/128, A61B 17/00

(54) **SURGICAL CLIP APPLIER**

(30) Priority: 17.08.2020 KR 20200102913
(71) Applicant: Hwainmedi Co., Ltd., Chungcheongnam-do 31094 (KR)
(72) Inventor: LEE, Seoung Won, Cheonan-si, Chungcheongnam-do (KR)
(74) Representative: BCKIP

(57) **Abstract**

The present invention relates to a surgical clip applier configured to launch a clip for ligating blood vessel or tubular tissue, the surgical clip applier including: a handle unit; an operating unit detachably installed and coupled to one side of the handle unit; a shaft unit detachably installed and coupled to one side of the handle unit while penetrating the operating unit; a ligation unit provided at one side of the shaft unit and configured to ligate the clip; and a storage unit detachably installed and coupled inside the handle unit, in which the clip is moved to a launch position of the ligation unit by pneumatic pressure in the storage unit, and in which only the shaft unit installed and coupled to one side of the handle unit is replaced in accordance with types of clips to be launched through the ligation unit, and the handle unit is used in common.

## Description

### TECHNICAL FIELD

The present invention relates to a surgical clip applier, and more particularly, to a surgical clip applier, in which a clip is moved to a launch position of a ligation unit and automatically loaded by pneumatic pressure in a storage unit, such that it is possible to achieve convenience of a patient and a user by improving stability and accuracy in launching the clip, and only a shaft unit installed and coupled to one side of a handle unit is replaced in accordance with the types of clips to be launched through the ligation unit, and the handle unit is used in common, such that it is possible to reduce operation costs and prevent waste of resources.

### BACKGROUND ART

During a surgical procedure, a practitioner should close or ligate blood vessels and other conduits at a surgical site prior to incision in order to prevent excessive bleeding in the patient and reduce the risk of other complications caused by infection. In addition, during a surgical procedure such as resection of an organ, a procedure of cutting a vein or artery or a procedure of hemostasis is frequently performed. In particular, in order to prevent excessive bleeding during a procedure of cutting the blood vessel, an operation of stopping bleeding is performed on both sides of the blood vessel to be cut before cutting the blood vessel.

In general, hemostasis of blood vessels is performed by suturing or ligating the blood vessels using surgical threads. However, in order to perform the hemostasis of the blood vessel using the surgical thread as described above, it is necessary to manually suture or ligate the blood vessel using needles and suture materials, which causes a problem in that a large amount of time is required to perform the surgical procedure such as endoscopic surgery which is limited in space and time, and it is difficult to perform the surgical procedure. In addition, there is always the likelihood of damage to blood vessels during the vascular ligation using the surgical thread or the likelihood of detachment due to loosening or slipping of the ligated surgical thread. In particular, this likelihood is increased in a larger blood vessel and may cause serious problems of massive bleeding after surgery.

Meanwhile, in order to solve the problem caused by using the surgical thread as described above, a surgical clip has been proposed and widely used. The above-mentioned surgical clip may be applied relatively easily and quickly in comparison with the method using the surgical thread, so the use of the surgical clip is increasing not only in open type surgery but also in endoscopic surgery, and a surgical clip applier for launching the clip is also being developed in various ways.

As such, recently, ligation methods using the clips made of various materials are widely used, and various surgical clips for this purpose have been proposed. In addition, the types of surgical clips are classified into a titanium clip and a polymer clip in accordance with the purposes.

However, in the case of the surgical clip applier in the related art, a trigger needs to be finely and precisely adjusted to load the clip to a launch position and sequentially launch the clips one by one. When the surgical clip applier is operated by a practitioner with low skill or the clip is loaded and launched by a mechanical mechanism, a defect may occur or the clip is not smoothly launched or loaded because of the manipulation by the mechanical mechanism and the configuration including a plurality of devices, such that several clips are launched at once, which causes a problem in that a device defect occurs, stability and accuracy deteriorate, and reliability is decreased.

Because the surgical clip applier in the related art cannot automatically load the clip to the launch position, reliability is decreased, accuracy of an operation deteriorates, and operating time is increased, which causes a problem in that a patient's recovery is decreased, a practitioner's fatigue is increased, and the patient's life is threatened because of an increase in medical accidents.

In the case of the surgical clip applier in the related art, only the surgical clip applier dedicated to launching the titanium clip is used to launch the titanium clip, and only the surgical clip applier dedicated to launching the polymer clip is used to launch the polymer clip. That is, because the titanium clip and the polymer clip have a large difference in their characteristics and advantages and disadvantages, the mechanisms and configurations for launching the titanium clip and the polymer clip are different from each other. Because different configurations need to be provided, that is, because the surgical clip applier configured to launch the titanium clip launches only the titanium clip, and the surgical clip applier configured to launch the polymer clip launches only the polymer clip. For this reason, a practitioner needs to purchase or use the independent surgical clip appliers to use the titanium clip and the polymer clip, which causes problems in that operation costs are increased, a patient's economic burdens are increased, inconvenience is caused to the practitioner, and waste of resources occurs.

In the case of the surgical clip applier in the related art, because the respective components or configurations such as a shaft and a handle unit are not optimized, assembled, and produced in modularized forms, manufacturing costs and manufacturing time are increased, and miniaturization cannot be achieved. For this reason, there is a problem in that the equipment becomes large, which causes inconvenience to a user or operator, and increases the storage and transport costs, and ultimately increases the burden of the patient's operating expenses.

### [Documents of Related Art]

### [Patent Documents]

(Patent Document 1) Korean Patent Application Laid-Open No. 10-2012-0018788
(Patent Document 2) Korean Patent Application Laid-Open No. 10-2012-0028911
(Patent Document 3) Korean Patent Application Laid-Open No. 10-2015-0112760
(Patent Document 4) Korean Patent Application Laid-Open No. 10-2020-0073008

### SUMMARY OF THE INVENTION

The present invention has been made in an effort to provide a surgical clip applier, in which a clip is moved to a launch position of a ligation unit and automatically loaded by pneumatic pressure in a storage unit, such that it is possible to achieve a user's convenience by improving stability and accuracy in launching the clip and maximizing reliability, and it is possible to shorten a patient's recovery by shortening the operating time, in which only a shaft unit installed and coupled to one side of a handle unit is replaced in accordance with the types of clips classified into a titanium clip and a polymer clip to be launched through the ligation unit, and the handle unit is used in common, and only a cartridge charged with the clips and the clip or the storage are replaced, such that as the handle unit is repeatedly used, and as a result, it is possible to reduce operation costs and prevent waste of resources, it is possible to reduce manufacturing costs and manufacturing time by improving assembly properties and productivity, it is possible to achieve convenience for storage and transport by making the surgical clip applier compact, and as a result, it is possible to maximize a consumer's satisfaction by reducing manufacturing costs.

In order to achieve the above-mentioned object, an exemplary embodiment of the present invention provides a surgical clip applier configured to launch a clip for ligating blood vessel or tubular tissue, the surgical clip applier including: a handle unit; an operating unit detachably installed and coupled to one side of the handle unit; a shaft unit detachably installed and coupled to one side of the handle unit while penetrating the operating unit; a ligation unit provided at one side of the shaft unit and configured to ligate the clip; and a storage unit detachably installed and coupled inside the handle unit, in which the clip is moved to a launch position of the ligation unit by pneumatic pressure in the storage unit, and in which only the shaft unit installed and coupled to one side of the handle unit is replaced in accordance with types of clips to be launched through the ligation unit, and the handle unit is used in common.

In another exemplary embodiment of the surgical clip applier according to the present invention, when the clip to be launched through the ligation unit is a titanium clip, a titanium clip shaft unit may be replaceably mounted on the handle unit and may have a cartridge which stores the plurality of the titanium clips and is detachably installed and coupled to a space portion formed at one side of the shaft unit adjacent to the ligation unit, and when the clip to be launched through the ligation unit is a polymer clip, a polymer clip shaft unit may be replaceably mounted on the handle unit so that the plurality of polymer clips is provided in the shaft unit and disposed in a horizontal direction of the shaft so as to be sequentially launched.

In still another exemplary embodiment of the surgical clip applier according to the present invention, the handle unit may include: a base part extending in the horizontal direction; a grip part disposed at a lower side of the base part, extending in a vertical direction, and having a trigger provided at one side of the grip part; and a tilting part tiltably installed at the other side of the grip part, in which the tilting part may include: a receiving part having a part turnably installed and coupled to the grip part and configured to receive the storage unit; and a link part connected, at one end thereof, to the receiving part and connected, at the other end thereof, to the tilting part, the link part being configured to tilt the receiving part in conjunction with the operation of turning the tilting part.

In yet another exemplary embodiment of the surgical clip applier according to the present invention, the base part may include: a cylinder part including a cylinder and a discharge part penetrating the cylinder in the horizontal direction, the cylinder part being configured to move forward or rearward in the horizontal direction at the other side in the base part; a flow path part communicating, at one side thereof, with the discharge part and communicating, at the other side thereof, with the storage unit received in the receiving part, the flow path part being penetratively formed to be inclined downward in the vertical direction at the other side in the base part in order to move the clip to the launch position of the ligation unit by the pneumatic pressure discharged from the storage unit; and a slide part having a rod part configured to move forward or rearward in the horizontal direction in conjunction with the horizontal forward/rearward movement of the cylinder part, the slide part being installed at one side in the base part so as to be adjacent to the cylinder part, and in which the grip part may include: an operating part installed in the grip part so as to be turned in a direction opposite to the turning direction of the trigger; a bridge part installed adjacent to the operating part in the grip part so as to be turned in the direction opposite to the turning direction of the operating part; a stopper part configured to move upward or downward in conjunction with the turning direction of the bridge part to block or allow the horizontal forward/rearward movement of the cylinder part; and a pressing part configured to elastically press the bridge part.

According to the surgical clip applier according to the present invention, the clip is moved to the launch position of the ligation unit and automatically loaded by the pneumatic pressure in the storage unit, such that it is possible to maximize reliability by improving stability and accuracy in launching the clip regardless of the skill of the practitioner who is the user.

According to the surgical clip applier according to the present invention, the clip is moved to the launch position of the ligation unit and automatically loaded by the pneumatic pressure in the storage unit, such that it is possible to achieve the user's convenience by improving stability and accuracy in launching the clip regardless of the skill of the practitioner, it is possible to achieve the patient's quick recovery by shortening operating time, and it is possible to maximize the patient's satisfaction by preventing a medical accident.

According to the surgical clip applier according to the present invention, only the shaft units, which are installed and coupled to one side of the handle unit, are changed in accordance with the types of clips classified into the titanium clips and the polymer clips to be launched through the ligation unit, and the handle unit is used in common, such that the handle unit may be repeatedly used by changing the storage for storing the pneumatic pressure and changing the clip or the cartridge, and as a result, it is possible to reduce operation costs and prevent waste of resources.

The respective components of the surgical clip applier according to the present invention are modularized, individually produced, and formed to be easily attached, detached, and assembled, and as a result, it is possible to reduce manufacturing costs and manufacturing time by improving assembly properties and productivity, it is possible to achieve convenience of storage and transport by making the surgical clip applier compact, it is possible to maximize the consumer's satisfaction and expand medical benefits by reducing manufacturing costs of the surgical clip applier.

### BRIEF DESCRIPTION OF THE DRAWINGS

FIG. 1 is a perspective view of a surgical clip applier according to the present invention which is configured to launch a titanium clip.
FIG. 2 is a cross-sectional view taken along line A-A in FIG. 1.
FIG. 3 is a perspective view of the surgical clip applier according to the present invention which is configured to launch a polymer clip.
FIG. 4 is a cross-sectional view taken along line B-B in FIG. 3.
FIGS. 5 to 7 are conceptual views for explaining an operating process of mounting a pneumatic tank in a handle unit of the surgical clip applier according to the present invention.

### DETAILED DESCRIPTION

Hereinafter, a surgical clip applier according to one exemplary embodiment of the present invention and a surgical clip applier according to another exemplary embodiment of the present invention will be described in detail with reference to the drawings. The following exemplary embodiments are provided as examples for fully transferring the spirit of the present invention to those skilled in the art. Therefore, the present invention is not limited to the exemplary embodiments described below and may be specified as other aspects. Further, in the drawings, a size and a thickness of the apparatus may be exaggerated for convenience. Like reference numerals indicate like constituent elements throughout the specification.

Advantages and features of the present invention and methods of achieving the advantages and features will be clear with reference to exemplary embodiments described in detail below together with the accompanying drawings. However, the present invention is not limited to the exemplary embodiments disclosed herein but will be implemented in various forms. The exemplary embodiments of the present invention are provided so that the present invention is completely disclosed, and a person with ordinary skill in the art can fully understand the scope of the present invention. The present invention will be defined only by the scope of the appended claims. Like reference numerals indicate like constituent elements throughout the specification. In the drawings, sizes and relative sizes of layers and regions may be exaggerated for clarity of description.

The terms used in the present specification are for explaining the exemplary embodiments, not for limiting the present invention. Unless particularly stated otherwise in the present specification, a singular form also includes a plural form. The terms such as "comprise (include)" and/or "comprising (including)" used in the specification do not exclude presence or addition of one or more other constituent elements, steps, operations, and/or elements, in addition to the mentioned constituent elements, steps, operations, and/or elements.

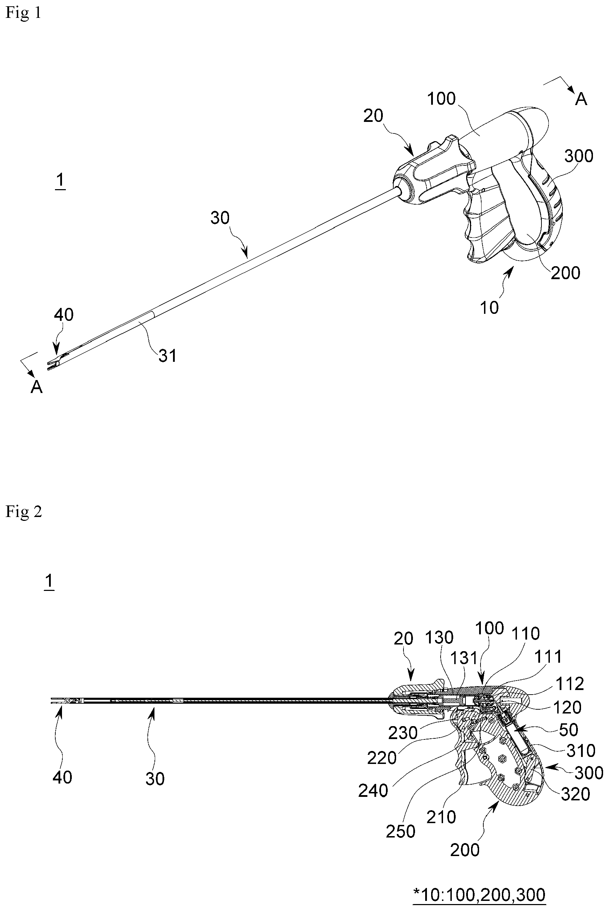

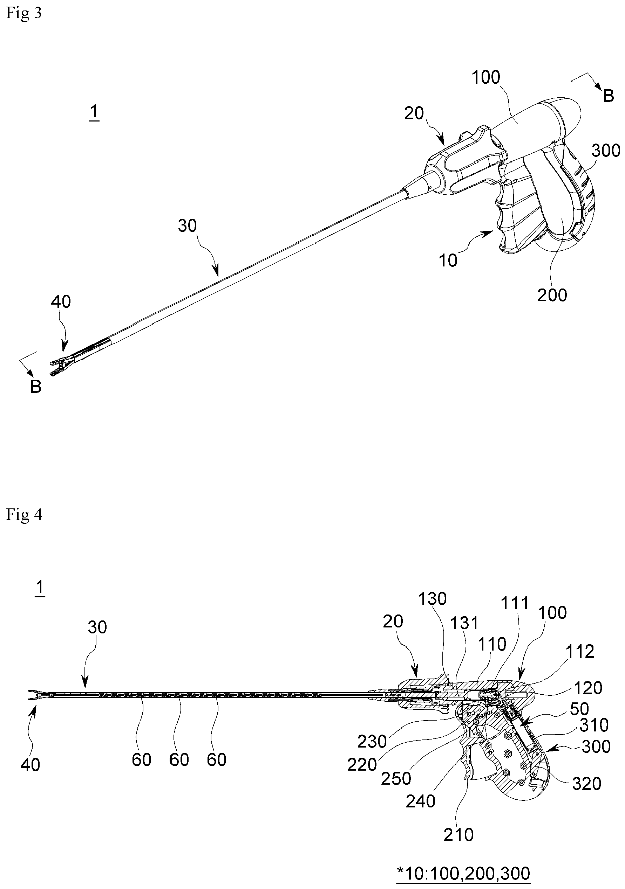

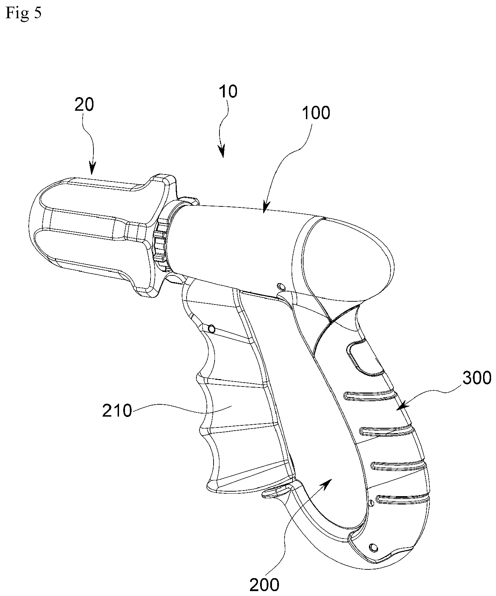

FIG. 1 is a perspective view of a surgical clip applier according to the present invention which is configured to launch a titanium clip, and FIG. 2 is a cross-sectional view taken along line A-A in FIG. 1. FIG. 3 is a perspective view of the surgical clip applier according to the present invention which is configured to launch a polymer clip, and FIG. 4 is a cross-sectional view taken along line B-B in FIG. 3. FIGS. 5 to 7 are conceptual views for explaining an operating process of mounting a pneumatic tank in a handle unit of the surgical clip applier according to the present invention.

The terms used below are defined as follows. The "one side" means a left side of each constituent member illustrated in FIGS. 1 to 7, and the term "the other side" means a side opposite to one side of the same member, that is, a right side of each of the members illustrated in FIGS. 1 to 7. In addition, the term "one end" means an upper side of each of the constituent members illustrated in FIGS. 1 to 7, and the term "the other end" means a side opposite to one end of the same member, that is, a lower side of each of the members illustrated in FIGS. 1 to 7. If a handle unit is positioned at the left side unlike the configuration illustrated in FIGS. 1 to 7, one side and the other side are defined in an opposite manner. The term "horizontal direction" means a horizontal direction on the same member, that is, a direction from the right side to the left side or from the left side to the right side in FIGS. 1 to 7, and the term "vertical direction" means a vertical direction on the same member which is perpendicular to the horizontal direction, that is, a height direction from the lower side to the upper side or from the upper side to the lower side in FIGS. 1 to 7. In addition, the term "upward (upper)" means a "vertically upward direction", that is, a direction toward the upper side in FIGS. 1 to 7, and the term "downward (lower)" means a "vertically downward direction", that is, a direction toward the lower side in FIGS. 1 to 7. In addition, the term "inside (inner portion)" means an inner portion included in an interface of the same member and relatively close to a center of the same member, that is, an inner side of each of the components illustrated in FIGS. 1 to 7, and the term "outside" means an outer side of the same member or each of the components relatively distant from the center of the same member.

A surgical clip applier 1 according to the present invention will be described with reference to FIGS. 1 to 7. As illustrated in FIGS. 1 to 7, a surgical clip applier 1 according to the present invention includes a handle unit 10, an operating unit 20, a shaft unit 30, a ligation unit 40, a storage unit 50, and a clip (a cartridge configured to store the clip, as necessary).

The handle unit 10 defines an external shape of the surgical clip applier. The handle unit 10 is formed in the form of a gun as a whole, but the present invention is not necessarily limited thereto. A base part 100, a grip part 200, and a tilting part 300 of the handle unit 10 may maintain rigidity and may be integrally formed to reduce manufacturing costs and manufacturing time. In addition, the handle unit 10 may be made of metal or plastic. The handle unit 10 serves as a handle so that a surgeon or a practitioner, who is a user, may hold and manipulate the surgical clip applier during a surgical procedure.

The operating unit 20 is detachably installed and coupled to one side of the handle unit 10. That is, the practitioner, who is a user, may rotate the operating unit clockwise or counterclockwise, the shaft unit thus rotates clockwise or counterclockwise in a direction identical to the rotation direction of the operating unit in conjunction with the operating unit, and the ligation unit provided at one side of the shaft unit thus rotates clockwise or counterclockwise in the direction identical to the rotation direction of the operating unit in conjunction with the shaft unit, such that the user may easily manipulate and change a launch position of a clip.

The shaft unit 30 is detachably installed and coupled to one side of the handle unit 10 while penetrating the operating unit 30.

The ligation unit 40 is provided at one side of the shaft unit and ligates the clip. That is, the ligation unit 40 includes a pair of jaws which is disposed to face each other and retracted inward or spread outward when operated by a trigger, such that the two jaws launch the clip while ligating blood vessel or tubular tissue with the clip.

The storage unit 50 is detachably installed and coupled inside the handle unit 10. The storage unit is configured as a pneumatic tank, but the present invention is not necessarily limited thereto. The pneumatic tank is charged with CO₂ gas, and a capacity of the pneumatic pressure gas stored in the storage unit may be variously set in accordance with the number of times the clips are loaded. That is, capacities of the pneumatic tank, which is the storage unit to be charged with CO₂, may be set such that the clips are loaded 5 times, 10 times, 12 times, 15 times, or 20 times. In addition, when the overall pneumatic pressure in the storage unit 50 is used, the surgical clip applier may be used after conveniently replacing only the storage unit. Further, the recovered storage unit may be recharged with CO₂ gas, sterilized, and then reused. As a result, it is possible to prevent waste of resources, reduce manufacturing costs and maintenance costs, achieve miniaturization, allow a surgical procedure to be quickly performed to shorten a patient's recovery time, and maximize a practitioner's convenience.

The clip is moved to the launch position of the ligation unit by the pneumatic pressure in the storage unit. That is, the storage unit 50 is received in a receiving part of the tilting part of the handle unit and stores pneumatic pressure which is power for automatically moving the clip to the launch position of the ligation unit by the operation of the trigger.

According to the surgical clip applier according to the present invention, only the shaft units, which are installed and coupled to one side of the handle unit, are changed in accordance with the types of clips to be launched through the ligation unit, and the handle unit is used in common.

That is, in a case in which the clip to be launched through the ligation unit is a titanium clip, a titanium clip shaft unit 30 is replaceably mounted on the handle unit 10, and the titanium clip shaft unit 30 has a cartridge which stores a plurality of titanium clips and is detachably installed and coupled to a space portion 31 formed at one side of the shaft unit 30 adjacent to the ligation unit 40. In a case in which the clip to be launched through the ligation unit 40 is a polymer clip, a polymer clip shaft unit 30 is replaceably mounted on the handle unit 10 while penetrating the operating unit 20, and a plurality of polymer clips 60 may be provided in the shaft unit 30 and disposed in the horizontal direction of the shaft so as to be sequentially launched.

Specifically, as illustrated in FIGS. 1 and 2, in the case in which the clip to be launched through the ligation unit is the titanium clip, the titanium clips are stored in the cartridge (not illustrated in the drawings). The cartridge for storing the plurality of titanium clips is detachably and replaceably installed in the space portion 31. The other side of the titanium clip shaft unit 30 is inserted and installed so that the titanium clip shaft unit 30, which may store and launch the titanium clip, is replaceably installed at one side of the handle unit 10 while penetrating the operating unit 20.

As illustrated in FIGS. 3 and 4, in the case in which the clip to be launched through the ligation unit is the polymer clip, the plurality of polymer clips 60 is not stored in a cartridge unlike the titanium clip, but the plurality of polymer clips 60 may be provided in the shaft unit 30 and disposed in a line in the horizontal direction of the shaft unit so as to be sequentially launched. The other side of the polymer clip shaft unit 30 is inserted and installed so that the polymer clip shaft unit 40, which may store and launch the plurality of polymer clips 60, is replaceably installed at one side of the handle unit 10 while penetrating the operating unit 20.

Therefore, according to the surgical clip applier according to the present invention, the clip is moved to the launch position of the ligation unit and automatically loaded by the pneumatic pressure in the storage unit, such that it is possible to maximize reliability by improving stability and accuracy in launching the clip regardless of the skill of the practitioner who is the user, it is possible to achieve the user's convenience and achieve a patient's quick recovery by shortening an operating time, and it is possible to improve the patient's satisfaction by preventing a medical accident. Further, only the shaft units, which are installed and coupled to one side of the handle unit, are changed in accordance with the types of clips classified into the titanium clips and the polymer clips to be launched through the ligation unit, and the handle unit is used in common, such that the handle unit may be repeatedly used by changing the storage for storing the pneumatic pressure and changing the clip or the cartridge, and as a result, it is possible to reduce operation costs and prevent waste of resources.

As illustrated in FIGS. 1 to 7, the handle unit 10 of the surgical clip applier 1 according to the present invention includes the base part 100, the grip part 200, and the tilting part 300.

The base part 100 extends in the horizontal direction and has therein a space in which a cylinder part, a flow path part, and a slide part, which will be described below, are installed.

The grip part 200 is disposed at the lower side of the base part and extends in the vertical direction. The grip part 200 has the trigger provided at one side of the grip part. That is, one end of the trigger is turnably coupled to a part of the grip part, such that the trigger is turned as the practitioner pulls the other end of the trigger. The flow path part is opened by the operation of turning the trigger, and the pneumatic pressure stored in the storage unit is discharged through a discharge part as the flow path part is opened. Therefore, a rod part is moved, and a push rod of the shaft unit is moved in conjunction with the movement of the rod part, such that the clip is moved to the launch position of the ligation unit.

The tilting part 300 is tiltably installed at the other side of the grip part. That is, as illustrated in FIGS. 5 to 7, when the user tilts the tilting part 300 clockwise, the tilting part is opened, such that the receiving part is tilted by a link part so as to be visible, and as a result, the replacement of the storage unit is easily performed.

As illustrated in FIGS. 1 to 7, the tilting part 300 of the handle unit 10 of the surgical clip applier 1 according to the present invention includes a receiving part 310 and a link part 320.

A part of the receiving part 310 is turnably installed and coupled to the grip part 200, such that the receiving part 310 receives the storage unit 50. That is, one end of the receiving part is turnably installed and coupled to the grip part, and the receiving part has a receiving space penetrating the receiving part, such that the receiving part 310 receives and stores the storage unit 50.

The link part 320 is connected, at one end thereof, to the receiving part 310 and connected, at the other end thereof, to the tilting part 300 to tilt the receiving part in conjunction with the operation of turning the tilting part, such that it is possible to reduce replacement time and operating time by allowing the storage unit to be easily replaced, and it is possible to maximize the satisfaction of the patient and the user by allowing the patient to be appropriately recovered.

As illustrated in FIGS. 1 to 7, the base part 100 of the handle unit 10 of the surgical clip applier 1 according to the present invention includes the cylinder part 110, the flow path part 120, and the slide part 130.

The cylinder part 110 moves forward or rearward in the horizontal direction at the other side in the base part. In addition, the cylinder part 110 has a cylinder installed in the cylinder part, and the discharge part formed to penetrate the cylinder in the horizontal direction.

The flow path part 120 communicates, at one side thereof, with the discharge part and communicates, at the other side thereof, with the storage unit received in the receiving part. The flow path part is penetratively formed to be inclined downward in the vertical direction at the other side in the base part so that the pneumatic pressure discharged from the storage unit moves the clip to the launch position of the ligation unit. That is, the storage unit is opened as the practitioner turns the trigger, and thus the pneumatic pressure discharged from the storage unit is transmitted to the discharge part through the flow path part 120. In addition, the flow path part is formed to penetrate the base part and inclined downward in the vertical direction at the other side in the base part so that the storage unit and the flow path part communicate with each other through the receiving part of the tilting part and the link structure of the link part in order to allow the storage unit to be easily replaced, such that a size of the base part may be minimized, and the receiving part of the tilting part and the link part are disposed at optimum positions, and as a result, it is possible to make the base part compact, it is possible to maintain rigidity while reducing manufacturing costs, it is possible to maintain rigidity while making the surgical clip applier compact, and it is possible to improve stability, accuracy, and reliability.

The slide part 130 has therein a rod part 131 that moves forward or rearward in the horizontal direction in conjunction with the horizontal forward/rearward movement of the cylinder part, and the slide part is installed at one side in the base part so as to be adjacent to the cylinder part. As described above, since the slide part 130 has the rod part 131 and moves forward or rearward in the horizontal direction in conjunction with the cylinder part, the titanium clip and the polymer clip may be constantly operated by the operation of turning the trigger even when the titanium clip shaft unit or the polymer clip shaft unit is replaced, such that the pneumatic pressure discharged from the storage unit is constantly transmitted to the clip, thereby simultaneously moving the clip to the launch position and launching and ligating the clip by the operation of the trigger.

As illustrated in FIGS. 1 to 7, the grip part 200 of the handle unit 10 of the surgical clip applier 1 according to the present invention further includes an operating part 220, a bridge part 230, a stopper part 240, and a pressing part 250.

The operating part 220 is installed in the grip part so as to be turned in a direction opposite to the turning direction of the trigger.

The bridge part 230 is installed adjacent to the operating part in the grip part so as to be turned in the direction opposite to the turning direction of the operating part.

The stopper part 240 moves upward or downward in conjunction with the turning direction of the bridge part to allow or block the horizontal forward/rearward movement of the cylinder part.

The pressing part 250 elastically presses the bridge part. That is, the pressing part 250 includes a coil spring or a disc spring that elastically presses the bridge part 230 in the direction opposite to the turning direction of the trigger.

Therefore, the respective components of the surgical clip applier according to the present invention are modularized, individually produced, and formed to be easily attached, detached, and assembled, and as a result, it is possible to reduce manufacturing costs and manufacturing time by improving assembly properties and productivity, it is possible to achieve convenience of storage and transport by making the surgical clip applier compact, it is possible to maximize the consumer's satisfaction and expand medical benefits by reducing manufacturing costs of the surgical clip applier.

An operational principle of replacing the storage unit of the surgical clip applier according to the present invention and automatically loading the clip to the launch position of the ligation unit by using the pneumatic pressure from the storage unit will be described with reference to FIGS. 1 to 7. Further, a principle of replacing and operating the titanium clip shaft unit for launching the titanium clip and the polymer clip shaft unit for launching the polymer clip.

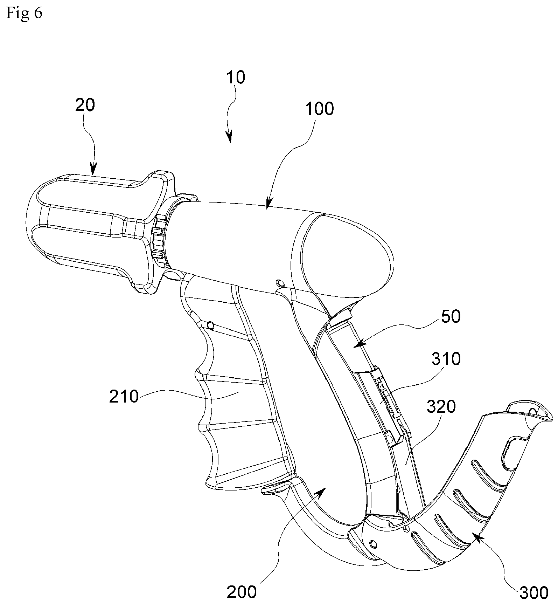

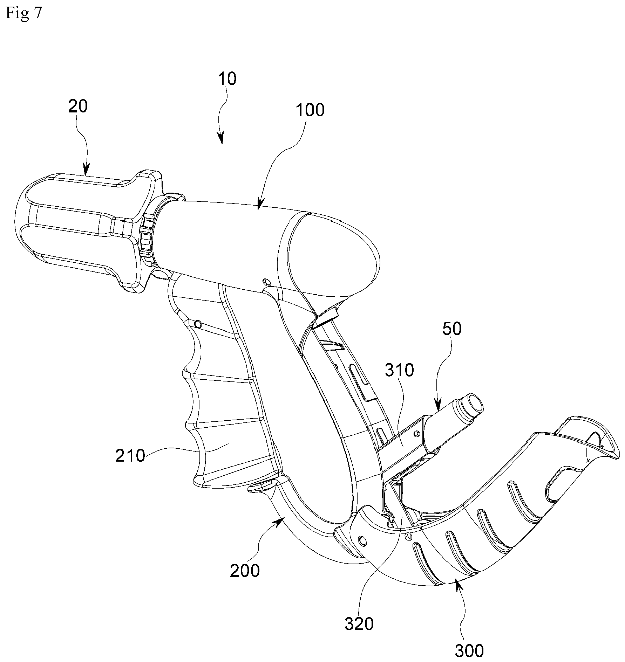

First, in order to automatically load the clip by using the pneumatic pressure, the storage unit needs to be mounted in the receiving part of the tilting part of the handle unit, and the storage unit from which the pneumatic pressure is completely discharged needs to be replaced. An operational principle of opening the receiving part of the tilting part will be described. In order to open the receiving part of the tilting part, when the user holds the tilting part in a state in which the tilting part is in contact with the grip part and the tilting part is closed as illustrated in FIG. 5, and then the user tilts the tilting part counterclockwise as illustrated in FIG. 5, the tilting part is tilted counterclockwise as illustrated in FIG. 6. When the tilting part is tilted counterclockwise as illustrated in FIG. 6, the link part, which is connected, at the other end thereof, to an inner surface of the tilting part, is tilted counterclockwise in conjunction with the tilting part, such that the receiving part connected to one end of the link part is tilted counterclockwise in conjunction with the link part. Therefore, as illustrated in FIG. 7, the receiving part and the tilting part are fully opened by the receiving part tilted counterclockwise by the link part. In this case, the user, as necessary, may remove and replace the storage unit from the receiving part with a new storage unit or may mount the storage unit in the vacant receiving part. Thereafter, when the user holds the tilting part and tilts the tilting part clockwise, the link part is tilted clockwise in conjunction with the tilting part, and at the same time, the receiving part is tilted clockwise and mounted in the grip part, and the tilting part comes into contact with the grip part and is closed again.

Therefore, according to the surgical clip applier according to the present invention, the clip is moved to the launch position of the ligation unit and automatically loaded by the pneumatic pressure in the storage unit, such that it is possible to maximize reliability by improving stability and accuracy in launching the clip regardless of the skill of the practitioner who is the user, it is possible to achieve the user's convenience and achieve a patient's quick recovery by shortening an operating time, and it is possible to improve the patient's satisfaction by preventing a medical accident.

A principle of replacing and operating the titanium clip shaft unit for launching the titanium clip and the polymer clip shaft unit for launching the polymer clip of the surgical clip applier according to the present invention will be described with reference to FIGS. 1 to 7.

The grip part has the operating part which is operated in the direction opposite to the turning direction of the trigger in conjunction with the turning direction of the trigger in accordance with the turning direction of the trigger, the bridge part which is turned in the direction opposite to the turning direction of the operating part, the bridge part which is moved upward or downward in conjunction with the turning direction of the bridge part, and the pressing part which allows the trigger to be always positioned at a basic position through the bridge part, thereby preventing the pneumatic pressure from leaking from the storage unit. When the trigger is turned counterclockwise by the user's manipulation, the operating part is turned clockwise in conjunction with the trigger, the bridge part is turned counterclockwise, the stopper part is moved downward, and the cylinder part is moved forward, such that the cylinder part opens the flow path part, the pneumatic pressure stored in the storage unit is discharged, the rod part is moved forward in conjunction with the discharge of the pneumatic pressure, the push rod is moved forward, and the clip is moved to the launch position and automatically loaded. As a result, the titanium clip and the polymer clip may be constantly operated by the operation of turning the trigger even when the titanium clip shaft unit or the polymer clip shaft unit is replaced, such that the pneumatic pressure discharged from the storage unit is constantly transmitted to the clip, thereby simultaneously moving the clip to the launch position and launching and ligating the clip by the operation of the trigger.

Therefore, according to the surgical clip applier according to the present invention, only the shaft units, which are installed and coupled to one side of the handle unit, are changed in accordance with the types of clips classified into the titanium clips and the polymer clips to be launched through the ligation unit, and the handle unit is used in common, such that the handle unit may be repeatedly used by changing the storage for storing the pneumatic pressure and changing the clip or the cartridge, and as a result, it is possible to reduce operation costs and prevent waste of resources. Further, the cylinder part, the grip part, and the tilting part of the handle unit, the operating unit, the titanium clip shaft unit having the ligation unit at one side thereof, the polymer clip shaft unit having the ligation unit at one side thereof, and the storage unit are individually produced as modules, to which some or all of these components are coupled, and formed to be easily attached, detached, and assembled, and as a result, it is possible to reduce manufacturing costs and manufacturing time by improving assembly properties and productivity, it is possible to achieve convenience of storage and transport by making the surgical clip applier compact, it is possible to maximize the consumer's satisfaction and expand medical benefits by reducing manufacturing costs of the surgical clip applier.

The handle unit, the operating unit, the shaft unit, the ligation unit, and the storage unit include a waterproof coating layer. In particular, the base part, the grip part, and the tilting part of the handle unit and the shaft unit include a waterproof coating layer, and the waterproof coating layer is formed using a waterproof coating composition. In addition, as another exemplary embodiment, a shrinkable film or a vacuum film may be used. In this case, the shrinkable film or the vacuum film may be made of PP, PE, or the like.

The waterproof coating composition may include a silane compound represented by the following Chemical Formula 1, a binder, inorganic particles, a surfactant, and a dispersant.

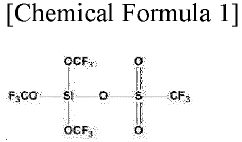

The binder may be selected from a group consisting of an acrylic-based binder, a urethane-based binder, a silicone-based binder, and a mixture thereof. More specifically, the binder is a silicone-based binder, and more specifically, polyhydrosiloxane may be used as the binder, but the present invention is not limited to this example.

The surfactant may be a betaine-based surfactant, and more specifically, the surfactant may be selected from a group consisting of alkyl betaine-based, amide betaine-based, sulfobetaine-based, hydroxysulfobetaine-based, amidosulfobetaine-based, phosphobetaine-based, and imidazoliniumbetaine-based surfactants, but the present invention is not limited to this example.

The dispersant may be made of a well-known component employed in the art as a dispersant for a carbon-based filler. More specifically, the dispersant may be selected from a group consisting of a polyester-based dispersant, a polyphenylene ether-based dispersant, a polyolefin-based dispersant, an acrylonitrile-butadiene-styrene copolymer dispersant, a polyarylate-based dispersant, a polyamide-based dispersant, a polyamide imide-based dispersant, a polyarylsulfone-based dispersant, a polyether imide-based dispersant, a polyether sulfone-based dispersant, a polyphenylene sulfide-based dispersant, a polyimide-based dispersant, a polyether ketone-based dispersant, a polybenzoxazole-based dispersant, a polyoxadiazole-based dispersant, a polybenzothiazole-based dispersant, a polybenzimidazole-based dispersant, a polypyridine-based dispersant, a polytrizaole-based dispersant, polypyrrolidine-based dispersant, a polydibenzofuran-based dispersant, a polysulfone-based dispersant, a polyurea-based dispersant, a polyurethane-based dispersant, polyphosphazene-based dispersant, and a mixture thereof, but the present invention is not limited to this example.

The inorganic particle is selected from a group consisting of titanium sol, alumina sol, silica sol, and a mixture thereof, and particularly, a mixture of alumina sol and silica sol may be used, but the present invention is not limited to this example.

More specifically, the coating composition includes a binder, and based on 100 part by weight of the binder, the coating composition may include 30 to 50 part by weight of silane compound represented by the following Chemical Formula 1, 5 to 10 part by weight of inorganic particles, 10 to 20 part by weight of a surfactant, and 10 to 15 part by weight of a dispersant. When constituent components of the coating composition within the above-mentioned ranges are used, a waterproof effect is exhibited by mixing actions between constituent components of the coating composition. A problem of a deterioration in waterproof effect may occur below or above the above-mentioned range.

The waterproof coating composition may form a waterproof coating layer by a spray coating method, but other well-known methods, instead of the spray coating method, may be used to form the waterproof coating layer by using the waterproof coating composition.

Therefore, the handle unit, the operating unit, the shaft unit, the ligation unit, and the storage unit include the waterproof coating layer. In particular, the base part, the grip part, and the tilting part of the handle unit and the shaft unit maximize waterproofness of the waterproof coating layer to protect the surgical clip applier from blood, foreign substances, cell sap, or other liquids produced while transporting, storing, or using the surgical clip applier or from moisture produced due to a difference in temperature or pressure in an operating room or a storage room, such that it is possible to improve corrosion resistance, durability, and lifespan of the surgical clip applier and to reduce manufacturing costs and storage costs by increasing transport and storage periods, it is possible to maximize safety, accuracy, and reliability of the surgical clip applier by improving stability of the surgical clip applier, and it is possible to prevent a medical accident or contamination caused by impurities.

While the present invention has been described above with reference to the exemplary embodiments of the present invention in the detailed description of the present invention, it may be understood, by those skilled in the art or those of ordinary skill in the art, that the present invention may be variously modified and changed without departing from the spirit and scope of the present invention disclosed in the claims. Accordingly, the technical scope of the present invention should not be limited to the contents disclosed in the detailed description of the specification but should be defined only by the claims.

## Claims

1. A surgical clip applier configured to launch a clip for ligating blood vessel or tubular tissue, the surgical clip applier comprising:
a handle unit;
an operating unit detachably installed and coupled to one side of the handle unit;
a shaft unit detachably installed and coupled to one side of the handle unit while penetrating the operating unit;
a ligation unit provided at one side of the shaft unit and configured to ligate the clip; and
a storage unit detachably installed and coupled inside the handle unit,
wherein the clip is moved to a launch position of the ligation unit by pneumatic pressure in the storage unit, and
wherein only the shaft unit installed and coupled to one side of the handle unit is replaced in accordance with types of clips to be launched through the ligation unit, and the handle unit is used in common.

2. The surgical clip applier of claim 1, wherein when the clip to be launched through the ligation unit is a titanium clip, a titanium clip shaft unit is replaceably mounted on the handle unit and has a cartridge which stores the plurality of the titanium clips and is detachably installed and coupled to a space portion formed at one side of the shaft unit adjacent to the ligation unit, and
wherein when the clip to be launched through the ligation unit is a polymer clip, a polymer clip shaft unit is replaceably mounted on the handle unit so that the plurality of polymer clips is provided in the shaft unit and disposed in a horizontal direction of the shaft so as to be sequentially launched.

3. The surgical clip applier of claim 2, wherein the handle unit comprises:
a base part extending in the horizontal direction;
a grip part disposed at a lower side of the base part, extending in a vertical direction, and having a trigger provided at one side of the grip part; and
a tilting part tiltably installed at the other side of the grip part, and
wherein the tilting part comprises:
a receiving part having a part turnably installed and coupled to the grip part and configured to receive the storage unit; and
a link part connected, at one end thereof, to the receiving part and connected, at the other end thereof, to the tilting part, the link part being configured to tilt the receiving part in conjunction with the operation of turning the tilting part.

4. The surgical clip applier of claim 3, wherein the base part comprises:
a cylinder part comprising a cylinder and a discharge part penetrating the cylinder in the horizontal direction, the cylinder part being configured to move forward or rearward in the horizontal direction at the other side in the base part;
a flow path part communicating, at one side thereof, with the discharge part and communicating, at the other side thereof, with the storage unit received in the receiving part, the flow path part being penetratively formed to be inclined downward in the vertical direction at the other side in the base part in order to move the clip to the launch position of the ligation unit by the pneumatic pressure discharged from the storage unit; and
a slide part having a rod part configured to move forward or rearward in the horizontal direction in conjunction with the horizontal forward/rearward movement of the cylinder part, the slide part being installed at one side in the base part so as to be adjacent to the cylinder part, and
wherein the grip part comprises:
an operating part installed in the grip part so as to be turned in a direction opposite to the turning direction of the trigger;
a bridge part installed adjacent to the operating part in the grip part so as to be turned in the direction opposite to the turning direction of the operating part;
a stopper part configured to move upward or downward in conjunction with the turning direction of the bridge part to block or allow the horizontal forward/rearward movement of the cylinder part; and
a pressing part configured to elastically press the bridge part.
